(19) Europäisches Patentamt
European Patent Office
Office européen des brevets

(11) Publication number: 0 055 668
A1

# (12) EUROPEAN PATENT APPLICATION

(21) Application number: 81402065.7

(22) Date of filing: 23.12.81

(51) Int. Cl.³: C 07 C 31/04
C 07 C 31/08, C 07 C 31/20
C 07 C 29/15

(30) Priority: 24.12.80 US 219873

(43) Date of publication of application:
07.07.82 Bulletin 82/27

(84) Designated Contracting States:
BE DE FR GB IT NL SE

(71) Applicant: UNION CARBIDE CORPORATION
Old Ridgebury Road
Danbury Connecticut 06817(US)

(72) Inventor: Dombek, Bernard Duane
1631 Woodvale Drive
Charleston Virginia 25314(US)

(72) Inventor: Warren, Barbara Knight
1620 Woodvale Drive
Charleston Virginia 25314(US)

(74) Representative: Rinuy, Guy et al,
14, Avenue de la Grande Armée
F-75017 Paris(FR)

## (54) Process for the production of ethylene glycol from synthesis gas in the presence of a ruthenium carbonyl complex.

(57) The present invention relates to a process for the production of ethylene glycol from synthesis gas in the presence of a ruthenium carbonyl complex.

In the process for the production of ethylene glycol by the reaction of a mixture of carbon monoxide and hydrogen (e.g., synthesis gas) in the presence of a ruthenium carbonyl complex, other products of reaction, such as methanol and ethanol, are obtained. This invention is concerned with decreasing the net production of one or more of such alcohols during the process. This is effected by providing to the reaction an additional amount of that alcohol of which it is desired to have the net production decreased.

EP 0 055 668 A1

Croydon Printing Company Ltd.

# PROCESS FOR THE PRODUCTION OF ETHYLENE GLYCOL FROM SYNTHESIS GAS IN THE PRESENCE OF A RUTHENIUM CARBONYL COMPLEX

This invention relates to an improvement in the process of making ethylene glycol by the reaction of carbon monoxide and hydrogen in the presence of a ruthenium carbonyl complex. The process of manufacturing ethylene glycol directly from the reaction of carbon monoxide and hydrogen in the presence of a ruthenium carbonyl complex is disclosed in co-pending US patent application Serial No. 091,242, filed November 15, 1979. One of the advantages of that process is its ability not only to produce very valuable ethylene glycol product but to also produce another valuable alcohol, ethanol. However, in the practice of such a process, it may be desired to change the efficiencies of the reaction towards the production of the various products. Ethylene glycol, without question, is the most valued of the alcohol products which the process of said co-pending application is capable of producing.

It is the purpose of this invention to enhance the effectiveness of the process of said co-pending US patent application, indeed of the processes which are generally described in the art for the manufacture of ethylene glycol utilizing ruthenium as the catalyst source, in which at least one alcohol, in most cases methanol, is simultaneously generated. The process of this invention involves providing, during the course of such reactions, an added amount of the particular alcohol whose net production it is desired to decrease. It also encompasses the concept of supplying mixtures of alcohol to the reaction for the purpose of decreasing the net production of the same

mixture of alcohol during the course of the reaction. The net effect of the process is a change in the ratio of ethylene glycol to added alcohol.

## DISCUSSION OF THE PRIOR ART

As indicated previously, co-pending US patent application Serial No. 091,242 describes the process for making ethylene glycol directly from synthesis gas utilizing a ruthenium carbonyl complex catalyst. The particular invention of said co-pending application is the ability of the process to manufacture methanol, ethylene glycol, and ethanol, or carboxylate derivatives thereof, directly from the reaction of hydrogen and carbon monoxide at moderate pressures when compared to the pressures which have been employed in the art prior thereto. The process of the co-pending application utilizes a solvent containing liquid phase comprising solubilized ruthenium carbonyl complex in which the solvent has a dielectric constant of at least 2, determined at 25°C. or at its melting point, whichever is higher; supplying hydrogen and carbon monoxide in such liquid phase; and maintaining the liquid phase for a sufficient period of time at a temperature and pressure which causes the hydrogen and carbon monoxide to react to produce the desired product. The temperature which is typically employed in the practice of that invention is between about 50°C. and 400°C. and the pressure is between about $35.15 \times 10^5$Pa (500 psia) and $1,054.6 \times 10^5$Pa (15,000 psia). The process of that application, in terms of selection of solvent,

temperature, and pressure, is equally applicable to the process of this invention.

There is disclosed in U.S. Patent Application Serial No. 921,698, filed July 3, 1978, in the name of John F. Knifton, assigned to Texaco Development Corp., a process for the production of alcohol and vicinal glycol esters from synthesis gas by reacting such synthesis gas in a carboxylic acid medium in the presence of a ruthenium catalyst at a temperature between about 100°C. and 350°C. and superatmospheric pressures of $35.15 \times 10^5$Pa (500 psia) or greater. In this particular application a co-catalyst is employed with the ruthenium catalytic species. The co-catalyst is selected from the group consisting of alkali metal salts, alkaline earth salts, quaternary ammonium salts, iminium salts and quaternary aliphatic phosphonium salts.

In U.S. Patent Application Serial No. 921,699, filed July 3, 1978 by Knifton, a similar process is described in which the catalyst contains either ruthenium or osmium. However, in this particular application the carboxylates of ethylene glycol are formed without the utilization of a co-catalyst. Essentially the same or similar disclosure as set forth in the aforementioned two patent applications can be found in U.S. Serial No. 967,943, filed December 11, 1978, and U.S. Serial No. 968,655, filed December 11, 1978. The disclosures of the aforementioned four patent applications can be found in British Patent Publication No. 2,024,811.

Pruett and Walker, U.S. Patent No. 3,833,634, patented September 3, 1974, based on an application originally filed December 21, 1971, describe a process for

preparing glycols by reacting an oxide of carbon with hydrogen using a rhodium carbonyl complex catalyst. The examples of the patent compare the reaction of hydrogen and carbon monoxide in the presence of the desired rhodium containing catalyst and other metals. In Example 9 of the patent, the reaction was attempted with triruthenium dodecacarbonyl as the catalyst using tetrahydrofuran as the solvent with a reaction temperature of 230°C., for 2 hours, and "the product contained no polyhydric alcohol." As shown in co-pending US patent application Serial No. 091,242 (see Example 82), Pruett and Walker apparently failed because they did not run at the conditions of reaction long enough and/or with enough ruthenium containing catalyst to achieve reaction to produce at least a detectable amount of a polyhydric alcohol, such as ethylene glycol. Example 82 employs substantially more ruthenium than did Pruett and Walker in their Example 9. Unquestionably, ruthenium is not as active a catalyst source to produce glycol as is rhodium under the conditions investigated.

Gresham, U.S. Patent No. 2,535,060 discloses a process for preparing monohydric alcohols by introducing carbon monoxide, hydrogen and a hydroxylated solvent into a reaction vessel and heating the mixture in the presence of a ruthenium containing substance and an alkaline reagent which controls the pH within the range of 7 to 11.5, at a temperature within the range of 150°C. to 300°C. under a pressure within the range of $200 \times 10^5$ to $1,000 \times 10^5$ Pa.

Solid ruthenium dioxide is used in Examples 1 and 2 of the Gresham patent.

At column 2, lines 30-33 of the patent, the patentee states his belief that ruthenium dioxide is reduced in situ during the reaction. Example 1 compares the use of a number of solutes such as phosphoric acid, acidic phosphate buffer, no solutes at all, ammonia and sodium bicarbonate. In this example, the solvent was water. In Example 2 of Gresham, a number of alcohols were characterized as solvents.

Gresham states that ruthenium and its compounds are "specific" in their effect upon this reaction and that other catalysts "do not lead to straight chain primary alcohols under the conditions of this process". There is no indication that Gresham's process, as operated by him, produced ethylene glycol.

Gresham's work should be contrasted with his earlier work described in U.S. Patent No. 2,636,046, filed October 16, 1948. In this patent, Gresham describes the production of polyfunctional oxygen-containing organic products including such compounds as ethylene glycol, glycerine, and the like. This is accomplished by the reaction of hydrogen with carbon monoxide in the presence of a solvent to produce glycol. According to this patent, the reaction of carbon monoxide with hydrogen must be at pressures of above 1,000 x $10^5$Pa (1,000 atmospheres) and "particularly above a minimum of about 1,400 atmospheres" in order to

obtain the "polyfunctional oxygen-containing organic compounds... in excellent yield" (column 2, lines 9-17). The patent specifically states at column 2, lines 37-43, that

> "[I]n the hydrogenation of oxides of carbon at pressures of 1,000 atmospheres and below, virtually no polyfunctional compounds are produced. At pressures above 1,000 atmospheres and especially at pressures of about 1,500 to 5,000 atmospheres, preferably 2,000 to 5,000 atmospheres, polyfunctional compounds are obtained."

Though the examples of the patent describe the use only of cobalt catalyst, the patentee, at column 3, line 61, indicates that the catalyst may contain "cobalt, ruthenium, etc." According to the patentee, the most outstanding results are obtained by using a catalyst containing cobalt, especially compounds of cobalt which are soluble in at least one of the ingredients of the reaction mixture.

According to Roy L. Pruett, Annals, New York Academy of Sciences, Vol. 295, pp. 239-248 (1977), at page 245, metals other than rhodium were tested to determine the production of ethylene glycol from mixtures of carbon monoxide and hydrogen. These metals include cobalt, ruthenium, copper, manganese, iridium and platinum. Of these metals, only cobalt was found to have a slight activity; British Patent No. 665,698, which corresponds generally to the last mentioned Gresham U.S. Patent, was cited. Pruett stated that such slight activity with cobalt was "qualitatively" in agreement with the results obtained by Ziesecke, 1952, Brennstoff-Chem, 33:385.

Prior to the filing of U.S Patent No. 2,535,060

and subsequent to the filing of U.S. Patent No. 2,636,046, there was filed on April 12, 1949, a commonly assigned application by Howk, et al. which issued as U.S. Patent No. 2,549,470 on April 17, 1951. The Howk, et al. patent is directed to a catalytic process for making monohydric straight chain alcohols and does not mention the production of ethylene glycol. The patent emphasizes the production of straight chain primary hydroxyalkanes having from 3 to 50 or more carbon atoms in the molecule. This, the patent states, is accomplished by introducing hydrogen, carbon monoxide and a hydroxylated solvent into a reaction vessel, and heating the mixture in the presence of a catalyst of the class consisting of ruthenium metal, ruthenium oxide and ruthenium carbonyl, at a pressure within the range of $200 \times 10^5$ to $1,000 \times 10^5$Pa and at a temperature within the range of 100°C. to 250°C. The liquid hydroxyl-containing reaction medium may be water or alcohol, preferably a primary hydroxyalkane having from 1-10 carbon atoms per molecule. According to the patentee, a substantial proportion of the reaction product usually consists of alcohols containing more than 6 carbon atoms per molecule. The patent goes on to state (column 1, line 50, et seq.):

> "The reaction products usually contain virtually no hydrocarbons, acids, esters, or branched-chain alcohols. These results were entirely unexpected, in view of the existing knowledge of the catalytic reaction between carbon monoxide and hydrogen in the presence of alcohols and Group VIII metal catalysts."

According to the Howk, et al. patent:

> "It should be emphasized here that, under the conditions of temperature, pressure and gas ratios just described, no reaction takes place between carbon monoxide and hydrogen

BAD ORIGINAL

> in a liquid medium (water or alcohol) if one of the common group VIII metals, such as cobalt or nickel, is used as the catalyst. This is evidenced by the fact that, using, for example, a cobalt catalyst, no significant drop in pressure is observed when carbon monoxide and hydrogen are contacted under the conditions recited. Ruthenium is thus unexpectedly different from these related metals." (Column 4, lines 19-30.)

The numbered examples indicate an apparent preference for making normal-monohydric alcohols, with the proportion of pentane soluble to pentane insoluble alcohol being at least 2:1. In one example, starting at the bottom of column 6 of Howk, et al., the solvent employed is characterized as a carboxylic acid or anhydride rather than the neutral hydroxylated solvents which were described in the other examples. This comparative example demonstrated that in a process operated at 200°C. for 18 hours using pressures maintained in the range of $300 \times 10^5$-$950 \times 10^5$Pa by repressurizing periodically with synthesis gas, there was produced a reaction product containing "a large quantity of wax." According to the author, 40.55 parts of esters boiling from 59°C. at atmospheric pressure to 150°C. at 116 millimeters pressure were obtained and this can be compared to the wax obtained in the amount of 37.06 parts. In that particular example, the patentee appears to have demonstrated that when one does not employ the hydroxylated solvent, the amount of wax essentially equals the amount of pentane soluble alcohol products obtained. This is supported by the statement at column 2 of Gresham U.S. 2,535,060 which refers to Howk, et al.

At column 3, lines 54 et seq., Howk, et al. describe the influence that pressure has on the course of

the reaction. According to Howk, et al. with pressures up to about 150 x $10^5$ Pa the reaction products are only hydrocarbons. This appears to be in accord with recent work described by Masters, et al. in German Patent Application (Offenlegungsschrift) 2,644,185*, based upon British priority application Specification No. 40,322-75, filed October 2, 1975. Masters, et al. obtained only hydrocarbons at such pressures using a ruthenium catalyst.

Fenton, U.S. Patent No. 3,579,566, patented May 18, 1971, is concerned with a process of reducing organic acid anhydrides with hydrogen in the presence of a Group VIII noble metal catalyst and a biphyllic ligand of phosphorus, arsenic or antimony. The process of Fenton bears a remarkable similarity to oxo processing conditions to produce aldehydes and alcohols (compare with Oliver, et al., U.S. Patent No. 3,539,634, patented November 10, 1970) except that Fenton fails to supply an olefinic compound to the reaction. In the Fenton reaction, an acid anhydride, such as acetic acid anhydride, is reduced to ethylidene diacetate in the presence of hydrogen or $CO/H_2$ and a rhodium halide or a mixture of palladium chloride and ruthenium trichloride catalyst, provided in combination with triphenylphosphine. Ethylene glycol diacetate is also observed.

---

* See Doyle, et al., J. of Organometallic Chem., 174, C55-C58 (1979), who conclude that the process characterized in the German Offenlegungsschrift involved a heterogeneous Fischer-Tropsch reaction.

Of particular significance is the fact that none of Fenton's examples produce a methyl ester, as are produced by the process of co-pending U.S. Patent Application Serial No. 971,667, discussed below and encompassed herein. Another point is that it is possible that Fenton's ethylidene diacetate can be converted to ethylene glycol diacetate under the conditions of example 1.

The following is believed to be a fair analysis of the aforementioned references, i.e., what they teach one skilled in the art and the direction that they could lead one in pursuit of whatever is their objectives:

(1) Gresham, U.S. Patent No. 2,636,046 states that at exceedingly high pressures in excess of $1,500 \times 10^5$ Pa (1,500 atmospheres), that is in excess of about 1,500 kg/$cm^2$, one can produce some glycol and glycol esters by the reaction of carbon-monoxide and hydrogen utilizing, most desirably, a cobalt catalyst although some undescribed ruthenium compound can be substituted for cobalt.

(2) The Pruett and Walker patent makes a showing in Examples 9 and 17 at columns 11 and 12, respectively, that the reaction of CO and $H_2$ in the presence of ruthenium carbonyl and cobalt carbonyl complexes operated at

about $1,335.8 \times 10^5$ - $1,757.7 \times 10^5$ Pa (19,000-25,000 pounds/in.$^2$) pressure will, in the case of ruthenium, produce no polyhydric alcohols and, in the case of cobalt, produce trace amounts of mono and diacetates of ethylene glycol. Thus, with respect to the cobalt catalyst a minimum pressure of about $1,335.8 \times 10^5$ Pa (19,000 psi) seems to be needed to make any glycol compound. In the case of ruthenium, the pressure at which glycol can be made from CO and $H_2$ had not been defined.

(3) Howk, et al. (U.S. Patent No. 2,549,470) who employ a lower pressure reaction than Gresham (U.S. 2,636,046) produce only monohydric alcohols from the reaction of CO and $H_2$ using a solid ruthenium catalyst. The maximum pressure for the Howk, et al. process is about $1,000 \times 10^5$ Pa (1,000 atmospheres). The reaction produces a spectrum of monohydric alcohols ranging from methanol to very high molecular weight alcohols, some alcohols containing up to 40 carbon atoms. The products are classified as pentane soluble materials and pentane insoluble materials. The pentane insoluble higher alcohols are characterized as waxes and less desirable than the pentane soluble alcohols. When Howk, et al. ran the reaction in acetic acid at a pressure ranging from $300 \times 10^5$ to $950 \times 10^5$ Pa, there was produced "a large quantity of wax together with a

liquid." The amount of wax was essentially the same amount, in parts by weight, as ester products, assumed to be esters of monohydric alcohols.

(4) The second Gresham Patent (U.S. Patent No. 2,535,060) appears to be an improvement on the Howk, et al. patent. It describes the desirability of controlling the pH of the reaction medium in the reaction between carbon monoxide and hydrogen in the presence of a ruthenium-containing catalyst, such as described by Howk, et al. The presence of trace amounts of carboxylic acid is considered very undesirable by Gresham. Gresham states that traces of carboxylic acids produce an acidity which "has a very profound effect upon the subsequent course of the reaction, causing the formation of relatively longer chain products, such as waxy alcohols containing up to 50 or more carbon atoms per molecule (c.f. co-pending application of Hager and Howk, Serial No. 87,114, filed April 12, 1949). If the pH is more strongly acidic, high molecular weight waxy products are formed in still greater proportions." The co-pending US patent application referred to is the Howk, et al., U.S. Patent No. 2,549,470, mentioned previously. Thus, Gresham specifies that it is desirable to maintain the pH of the reaction solution alkaline in order to

obtain a better distribution of straight chain monohydric primary alcohols. According to Gresham, the quantity of methanol formed in his reaction "is extremely small" (see column 1, line 49).

(5) There is apparently a minimum pressure according to Howk, et al. used to avoid the formation of hydrocarbons and this appears to be supported by the disclosure of Masters, et al., supra. However, in view of Doyle, et al., supra, there may be a greater similarity in the processes of Howk, et al. and Masters, et al.

(6) The choice of metal catalyst and the appropriate conditions for such kinds of reactions are not predictable. For example, Pruett, et al., the Gresham '060, Howk, et al., and Pruett, state that many metals do not function as catalysts in the reactions they are concerned with.

(7) Fenton utilized rhodium, palladium and ruthenium halides in the presence of a mixture of hydrogen and carbon monoxide and an acid anhydride, and recognized only the reduction of the anhydride.

In co-pending US patent application Serial No. 971,667, filed December 21, 1978, there is described a process for producing methyl and ethylene glycol esters by reacting carbon monoxide and hydrogen in a homogeneous liquid phase

mixture comprising a ruthenium carbonyl complex and acyl compound such as acetic acid. The reaction is effected at a temperature between about 50°C. to about 400°C. and a pressure of between about $35.15 \times 10^5$ Pa (500 psia) and about $878.84 \times 10^5$ Pa (12,500 psia) for a period of time sufficient to produce such esters as the predominant product.

In co-pending US patent application Serial No. 971,750, filed December 21, 1978, there is described an improved process for producing methyl and ethylene glycol esters as described in Serial No. 971,667, in which the improvement comprises maintaining the combined concentration of methyl ester, ethylene glycol ester and water in the reaction medium at less than about 30 vol. %.

In a recent report (J. Amer. Chem. Soc., <u>101</u>, 7419 (1979).), J.S. Bradley of Exxon Corporation reported the production of methanol and methyl formate at a selectivity greater than 99% without hydrocarbon products detected, by the reaction of synthesis gas ($H_2$:CO=3:2) under pressures on the order of 1,300 $\times 10^5$ Pa and at temperatures around 270°C. using a Ru catalyst. Bradley observed that no ethanol, ethylene glycol, or acetates formed. Compare this result with that found by Pruett and Walker, <u>supra</u>, and the work of Fonseca, et al. and Williamson, et al., <u>infra</u>.

An interesting exception to the previously reported inactivity of ruthenium catalyst to produce glycol is the high pressure (viz 1650 x $10^5$-1750 x $10^5$Pa) experiment reported by Fonseca, Jenner, Kiennemann, and Deluzarche, et al., High Pressure Science and Technology, 6th AIRAPT Conference (Chapt. "High Pressure Synthesis of Polyalcohols by Catalytic Hydrogenation of Carbon Monoxide"), pages 733-738 (1979), published by Plenum Press, New York (see also a discussion of the same work in Erdöl Und Kohle, 32, 313 (1979)). The authors report the reaction in tetraglyme of a $CO:H_2$ (1:2 ratio) mixture at 1650 x $10^5$-1765 x $10^5$Pa, i.e., about 25,000 psi (1,757.6 Kg/$cm^2$) and 230°C using triruthenium dodecacarbonyl and 2-pyridinol as a ligand both in unstated amounts, for a period of 5 hours. The authors report a percent conversion of 12.9 (unstated basis), and percent yield of polyols of 3 (unstated basis), and percent selectivities as follows: ethylene glycol, 22.9; glycerine, 0; methanol, 16.1 However, in a manuscript to be published entitled "Reactions $CO-H_2$ in Liquid Phase in Presence of Ruthenium Catalysts," by Jenner, Kiennemann, Bagherzadah, and Deluzarche, it is stated that with respect to the above experiment "We never could reproduce the run with $Ru_3(CO)_{12}$ when operating in a vessel which has not been in contact with any rhodium catalyst. We suspect that in the former run, the formation of ethylene glycol was due to catalysis with metallic sediments of rhodium encrusted on the wall of the vessel (we showed that ethylene glycol is produced in appreciable yield with rhodium foam)".*

---

*This report may be relevant to the reports by Williamson et al, (infra) and Keim et al, (infra).

In Williamson, et al., United States Patent 4,170,605 patented October 9, 1979 the patentees report in Examples I and II the reaction in 1-propanol of synthesis gas ($CO:H_2 = 1:1$) at $1720 \times 10^5$ Pa (25,000 psig) and at 230°C using ruthenium tris(acetylacetonate) and 2-hydroxypyridine, the latter being the same ligand employed by Fonseca, et al, supra, for a period of 2 and 3 hours, respectively. In Example 1, Williamson, et al., report the production of 4 grams of product** containing (mole percent basis) : ethylene glycol, 57; and methanol 25. In Example II, 7 grams of product** are reported containing 66 and 16 mole percent of ethylene glycol and methanol, respectively.

W. Keim, et al., (Journal of Catalysis, 61, 359 (1980)) has reported that reactions of $Ru_3(CO)_{12}$ under very high pressures $2{,}000 \times 10^5$ Pa (2,000 bars) produce mainly methanol and methyl formate, but traces of glycol (0.8 to 1.2 percent of the total products) were also seen. In one experiment a small amount of ethanol was detected. No glycerine was observed in these reactions.

As pointed out above, ethylene glycol can be produced directly from a mixture of hydrogen and carbon monoxide using a rhodium carbonyl complex as a catalyst. The literature describes (see U.S. Patent No. 3,957,857, issued May 18, 1976) that a desirable rhodium compound can

** Included in the 4 and 7 grams of product are trace amounts of water and methylformate as well as 16 mole percent (Example I) and 15 mole percent (Example II) of propylformate. The latter compound would appear to be derived from 1-propanol initially present in the reaction mixture, rather than a synthesis gas-derived product.

be in the form of a rhodium carbonyl cluster compound. There has been a substantial amount of work done on the formation of ethylene glycol from mixtures of hydrogen and carbon monoxide (see the list of patents and applications recited in footnoted Table A below*).

---

* TABLE A:

| | |
|---|---|
| U.S.P. 3,833,634 | Patented September 3, 1974 |
| U.S.P. 3,878,214 | Patented April 15, 1975 |
| U.S.P. 3,878,290 | Patented April 15, 1975 |
| U.S.P. 3,878,292 | Patented April 15, 1975 |
| U.S.P. 3,886,364 | Patented May 27, 1975 |
| U.S.P. 3,929,969 | Patented December 30, 1975 |
| U.S.P. 3,940,432 | Patented February 24, 1976 |
| U.S.P. 3,944,588 | Patented March 16, 1976 |
| U.S.P. 3,948,965 | Patented April 6, 1976 |
| U.S.P. 3,952,039 | Patented April 20, 1976 |
| U.S.P. 3,957,857 | Patented May 18, 1976 |
| U.S.P. 3,968,136 | Patented July 6, 1976 |
| U.S.P. 3,974,259 | Patented August 10, 1976 |
| U.S.P. 3,989,799 | Patented November 2, 1976 |
| U.S.P. 4,001,289 | Patented January 4, 1977 |
| U.S.P. 4,013,700 | Patented March 22, 1977 |
| U.S.P. 4,111,975 | Patented September 5, 1978 |
| U.S.P. 4,115,428 | Patented September 19, 1978 |
| U.S.P. 4,115,433 | Patented September 19, 1978 |
| U.S.P. 4,133,776 | Patented January 9, 1979 |

* TABLE A (Cont'd.)

| | |
|---|---|
| U.S.P. 4,151,192 | Patented April 24, 1979 |
| U.S.P. 4,153,623 | Patented May 8, 1979 |
| U.S.P. 4,162,261 | Patented July 24, 1979 |
| U.S.P. 4,180,517 | Patented December 25, 1979 |
| U.S.P. 4,188,335 | Patented February 12, 1980 |
| U.S.P. 4,191,701 | Patented March 4, 1980 |
| U.S.P. 4,197,253 | Patented April 8, 1980 |
| U.S.P. 4,199,520 | Patented April 22, 1980 |
| U.S.P. 4,199,521 | Patented April 22, 1980 |
| U.S.P. 4,211,719 | Patented July 8, 1980 |
| U.S.P. 4,224,235 | Patented September 23, 1980 |
| U.S.P. 4,224,237 | Patented September 23, 1980 |
| U.S.P. 4,225,530 | Patented September 30, 1980 |
| U.S.P. 4,228,094 | Patented October 14, 1980 |
| U.S. Ser. No. 618,021 | Filed September 30, 1975 |
| U.S. Ser. No. 862,554 | Filed December 20, 1977 |
| U.S. Ser. No. 882,396 | Filed March 1, 1978 |
| U.S. Ser. No. 919,419 | Filed June 27, 1978 |
| U.S. Ser. No. 946,313 | Filed September 27, 1978 |
| U.S. Ser. No. 946,314 | Filed September 27, 1978 |
| U.S. Ser. No. 958,383 | Filed November 7, 1978 |
| U.S. Ser. No. 146,211 | Filed May 5, 1980 |
| U.S. Ser. No. 144,048 | Filed April 28, 1980 |
| U.S. Ser. No. 62,357 | Filed July 31, 1979 |
| U.S. Ser. No. 70,003 | Filed August 27, 1979 |
| U.S. Ser. No. 71,576 | Filed August 31, 1979 |
| U.S. Ser. No. 81,919 | Filed October 4, 1979 |

The above discussion provides a detailed characterization of technology heretofore published or filed upon which relates to the direct production of ethylene glycol from mixtures of carbon monoxide and hydrogen or the production of monohydric alcohols from the direct reaction of hydrogen and carbon monoxide in the presence of a ruthenium catalyst. For the purposes of the discussion and descriptions contained herein, mixtures of hydrogen and carbon monoxide, regardless of the amount of each present, will be characterized, for the sake of convenience, as "synthesis gas". Thus, mole

---

*TABLE A (Cont'd.)

| | |
|---|---|
| U.S. Ser. No. 85,208 | Filed October 16, 1979 |
| U.S. Ser. No. 135,403 | Filed March 31, 1980 |
| U.S. Ser. No. 164,009 | Filed June 30, 1980 |
| U.S. Ser. No. 163,972 | Filed June 30, 1980 |
| U.S. Ser. No. 163,973 | Filed June 30, 1980 |
| U.S. Ser. No. 192,498 | Filed September 30, 1980 |

ratios of hydrogen to carbon monoxide of e.g. 40 to 1 and .05 to 1 are arbitrarily classified as "synthesis gas". Where the molar ratio of one to the other is significant to the invention herein described, then specific reference to the desired molar ratio will be made.

THE PROBLEM

Owing to the limited availability of petroleum sources the cost of producing chemicals from petroleum has been steadily increasing. Obviously a different low cost source is needed which can be converted into the valuable chemicals now derived from petroleum sources. Synthesis gas is one such source which can be effectively utilized in certain circumstances to make chemicals.

The most desirable aspect of synthesis gas is that it can be produced from non-petroleum sources. Synthesis gas is derived by the combustion of any carbonaceous material including coal, or any organic material, such as hydrocarbons, carbohydrates and the like. Synthesis gas has for a long time been considered a desirable starting material for the manufacture of a variety of chemicals. A number of chemicals have been made commercially from synthesis gas. Hydrocarbons have been made by the Fischer-Tropsch catalytic reaction. Methanol is

commercially manufactured by a heterogeneous catalytic reaction from synthesis gas. Aldehydes and alcohols are made from the reaction of olefins and synthesis gas. If one could expand the production of chemicals in a commercial manner from synthesis gas then one would not be as presently dependent upon petroleum as the basic raw material even though it is an excellent raw material for making synthesis gas.

There is described herein a process which is an improvement on previous processes for the production of a host of valuable chemicals generated by the reaction of synthesis gas with a ruthenium-based catalyst. The process of this invention, as well as others hereinbefore described involves the conversion of synthesis gas, however derived, into a limited variety of valuable alcohol compounds but is particularly concerned with the manufacture of ethylene glycol as distinguished from the other processes which include the generation, in considerable quantities, of monohydric alcohols. Contrary to the process described in US patent application Serial No. 091,242, the process of this invention decreases the production, overall, of large amounts of added alcohol. The net effect of the process is a change in the ratio of ethylene glycol to added alcohol (i.e. the net production of said added alcohol) with the resulting obvious advantages in relative amount of the products formed and in product separations.

One of the deficiencies of the aforementioned processes for making ethylene glycol from synthesis gas utilizing a rhodium carbonyl complex catalyst is the enormous price of rhodium. Rhodium presently is employed in catalytic converters which comprise the automotive combustion devices for reducing automotive pollutant emissions. The high cost of rhodium is created by its limited availability and the tremendous demand for it. Thus, a commercial process which uses rhodium as a catalyst is affected by the high capital expense to purchase the metal and the strict controls needed to limit catalyst losses in order to keep the economics of the process competitive.* Ruthenium, on the other hand, is a precious metal which has no significant commercial application. Its present cost is approximately 1/20th and less than that of rhodium even though its concentration in the ore from which both are obtained is about the same. Ruthenium has been explored as a catalyst by many. It has been considered as a hydrogenation catalyst, as a hydroformylation catalyst, as a catalyst to produce a wide range of monohydric alcohols (non-specific as to any of

---

* See Cornils, et al., Hydrocarbon Processing, June, 1975, pp. 83 to 91.

them) exclusive of methanol, as an alcohol homologation catalyst such as for the conversion of methanol to ethanol,* as a high pressure catalyst to selectively produce methanol and methyl formate, and its inactivity has been noted as a catalyst to produce glycol, see above.

## THE INVENTION

This invention relates to processes for selectively making ethylene glycol. This is achieved by the reaction of hydrogen and carbon monoxide in the presence of a ruthenium carbonyl complex. The reaction is generally effected in a homogeneous liquid phase comprising solubilized ruthenium carbonyl complex in a solvent having a dielectric constant of at least 2, determined at 25°C. or at its melting point, whichever is higher. To this solution is supplied hydrogen and carbon monoxide and they are maintained in the liquid phase for a sufficient period of time and pressure to cause the hydrogen and carbon monoxide to react to produce ethylene glycol. The reaction is effected at a temperature of between about 50°C. and 400°C. and the pressure may be as low as about $35.15 \times 10^5$ Pa (500 psia or 35.15 $kg/cm^2$) to $35.15 \times 10^5$ Pa (50,000 psia or 35.15 $kg/cm^2$). During the course of the reaction there is provided to the reaction mixture an effective amount of the

---

* See, for example, U.S. Patents 4,133,966 and 3,285,948; and Japanese Patent Application (Kokai) No. 52-73804/77 (June 21, 1977) [Application No. 50-149391/75 (application date, December 15, 1975)] to Mitsubishi Gas Chemical Industry Company.

particular alcohol, of which a net decrease in production of said corresponding alcohol is desired. For example, if one wishes to decrease the net production of methanol, then methanol is provided to the reaction. If the net production of ethanol is desired to be decreased, then ethanol should be provided to the reaction. If the net production of both methanol and ethanol are desired to be decreased, then a mixture of the two in appropriate amounts should be provided to the reaction.

It is believed that the net production of a particular alcohol is decreased either as a result of: 1) a decrease in the rate of production to alcohol; 2) conversion of the alcohol to other compounds; or a combination of 1) and 2). In certain instances it may be desirous to provide for the formation of these other compounds.

The term "net production", as used herein, describes the amount of measured alcohol in the process minus any amount of said alcohol added to the process. The use of the word "added" simply means that alcohol is provided to the process by one or more possible means, as hereinafter described. A decrease in the net production refers to a decrease in the amount of alcohol as determined from the alcohol in the process minus any added alcohol as compared to the amount of said alcohol which would have been produced were no alcohol added to the process.

A preferred and significant embodiment of this process involves the continuous production of ethylene glycol by the reaction of hydrogen and carbon monoxide as afore defined by continuously supplying to

the reaction either one or more of the alcohols whose net production it is desired to decrease. This can be done by recycling one or more of such alcohols which have been produced in the course of the reaction, or by introducing alcohol from some other source to the reaction.

FURTHER DISCUSSION OF THE INVENTION

This process constitutes a relatively low pressure process for selectively converting synthesis gas to such valuable chemicals as the alcohol products ethylene glycol, ethanol and methanol. Also produced by the process of this invention are glycerol (i.e. glycerine), 1,2-propylene glycol, 1-propanol and methyl formate. However, the process of this invention is mainly concerned with the production of ethylene glycol (the most valued product), ethanol and methanol since they are produced in significantly greater amounts than the other products. This process is capable of being oriented to enhance the selectivity in favor of any one of methanol, ethanol and ethylene glycol. An added feature of this invention may be the ability to enhance the productivity, when desired, of such by-products as glycerol. The process of this invention is accomplished even when

the predominant products of the reaction are derivatives such as methyl carboxylates, ethyl carboxylates and ethylene glycol mono- and dicarboxylates.

The process of this invention is generally carried out with the ruthenium carbonyl complex dissolved in a solvent, even though such complex may exist during the reaction in more than one phase. In this sense, the reaction is termed a homogeneous liquid phase reaction. There may be more than one phase existing in the reaction zone but the ruthenium carbonyl complex existing as the catalyst is always dissolved in at least one of such phases and is always in a dissolved liquid state. A problem with heterogeneous ruthenium catalysis in the reaction zone is that such will induce the Fischer-Tropsch reaction resulting in the formation of hydrocarbons and/or a variety of oxygenated hydrocarbons having a variety of molecular weights with low selectivity to any one compound. In fact, the presence of such products suggests that undissolved ruthenium is present.

Thus, the process of this invention involves the solubilization of ruthenium in the presence of synthesis gas at temperatures, pressures and for a period of time sufficient to produce ethylene glycol. Such conditions are set forth herein. In simplistic and in the broadest terms, the invention comprises the solubilization under the reaction conditions (i.e., time, temperature and pressure) of a ruthenium source, preferably ruthenium in

the absence of any other platinum group metal (viz., platinum, palladium, rhodium and iridium) , in an appropriate solvent, preferably one which has a dielectric constant of at least 2 determined at 25°C. or at its melting point, whichever is the higher value, under a prescribed synthesis gas pressure. The reaction conditions comprise (i) a period of time at a temperature and pressure which cause the hydrogen and carbon monoxide to react to produce the desired products, (ii) a temperature between about 50°C. and 400°C. and (iii) a pressure between $35.15 \times 10^5$ Pa (500 psia or 35.15 kg/$cm^2$) and $1,054.6 \times 10^5$ Pa (15,000 psia or 1,054.6 kg/$cm^2$). The catalyst of this invention is the ruthenium containing carbonyl complex which under the prescribed reaction conditions catalyzes the aforementioned reaction between carbon monoxide and hydrogen.

The process of this invention is distinctive in its preferred embodiment by the selection of materials which comprise the homogeneous liquid phase mixture such that these parameters are selected to maintain the stability of the ruthenium containing catalyst. According to the process of this invention the utilization of added alcohol decreases the net production of added alcohol and changes the ratio of ethylene glycol to said added alcohol.

In the preferred form of the invention the

process is carried out in the presence of a promoter. A promoter, in the context of this invention, is a material provided to the reaction which provides a promotional effect in that it enhances the production (viz., rate, yield or efficiency) of any of the products, or it improves the selectivity of the reaction toward ethylene glycol rather than methanol or ethanol, or it improves the selectivity of the reaction to ethanol rather than methanol irrespective of the amount of ethylene glycol produced, or it helps to reduce the loss of ruthenium during the reaction. The effectiveness of the promoter will in large measure depend upon the reaction conditions selected. Operation of the process in the absence of the promoter will result in most instances in less productivity and therefore, exploitation of the process in a commercial sense will probably necessitate the use of a promoter.

The amount of promoter added to the process is that amount which provides the promotional effect. The maximum amount employed is that amount whose presence is too costly for the economical operation of the process, or substantially reduces the promotional effect without any advantage, or provides no advantage in the operation of the process, or a combination of these factors. The promoter can be a material used in miniscule quantities to a material employed in maximum quantities, such as a solvent for the reaction and the ruthenium

carbonyl complex catalyst. Indeed, the promoter can be a material such as carboxylic acids, which when present react with the products of the reaction.

Apart from the conditions of the reaction in terms of time, temperature and pressure, the selection of the conditions of the reaction are not narrowly limited, as discussed herein, yet there may to some degree be cooperation in the various conditions such that each contributes to the success of the process and the selection of one oftentimes dictates the selection of the other in order to maximize the benefits of the invention. The selection of solvent and optionally the promoter constitute important considerations in the most advantageous practice of this invention. The selections of solvent and the promoter are not narrowly limited yet there appears to be some degree of cooperation that each imparts to the success of the process and the selection of one oftentimes dictates the selection of the other in order to maximize the benefits of the invention.

It is found necessary that there be used a solvent that is capable of maintaining the chosen ruthenium carbonyl complex and, optionally, the Lewis base promoter (if it is not the solvent), in the homogeneous liquid phase mixture throughout the reaction. This appears to be a prime function of the solvent. The solvent may possibly provide an additional benefit, such as influencing the kinds and extent of ion pairing that exist during the course of the reaction.

The catalyst of this invention is a ruthenium compound which contains carbon monoxide directly bonded to ruthenium (ruthenium carbonyl). The ruthenium compound which is provided to the reaction is not necessarily in a form which will effectively catalyze the reaction even if it contains a carbon monoxide ligand bonded to it. Ruthenium compounds such as ruthenium salts, oxides and carbonyl clusters may be introduced to the reaction in a condition which allows them to be solubilized, and under the conditions of the reaction they are converted into a carbonyl complex which effectively catalyzes the reaction. That is why they are defined in terms of products made by the process. The composition and structure of the ruthenium carbonyl complex which catalyzes the desired reaction are not specifically known. It may be a monoruthenium or polyruthenium compound. Illustrative of polyruthenium compounds are the well-known cluster compounds of ruthenium. However, the addition of a cluster containing only a carbonyl ligand such as $Ru_3(CO)_{12}$ does not alone create the catalyst and as such cause the catalytic reaction. Some modification of such structure is needed. Factors in achieving the catalyst are the reaction parameters, the choice of solvent and, optionally the promoter that one employs. Because varied reaction conditions and solvents, with and without promoters, result in different amounts of the desired products of the process, and different rates, efficiencies and/or yields, it is presumed that each provides a different and distinct catalytic environment.

The ruthenium-containing substances which may be employed in the practice of this invention to form the catalyst under process conditions encompass those which are described, for example, in Gresham, U.S. Patent No. 2,535,060, at column 2, starting at line 38 to line 48, and ruthenium carbonyl compounds. It is not advisable to place ruthenium compounds or substances on a support material for use in the process of this invention because such offers no benefits over solubilizing such ruthenium compounds in combination with the aforementioned solvent and Lewis base promoter. Moreover, ruthenium deposited on a support material can be expected to be solubilized in the homogeneous liquid phase reaction system of this invention as it is contacted with carbon monoxide. Even ruthenium metal in the presence of the solvent, carbon monoxide and hydrogen, can be converted to a ruthenium carbonyl complex which is soluble. Ruthenium oxides, such as dioxide, sesquioxide, or tetraoxide, are capable, under appropriate conditions, of being solubilized and converted to a carbonyl complex which can be used to form the catalyst under the conditions of this process. However, when using such insoluble ruthenium compounds, they must first be solubilized before the effective operation of the process of this invention. Ruthenium carbonyl compounds (which

include ruthenium carbonyl hydrides or ruthenium carbonyl clusters) are already provided with a carbonyl ligand, and under the conditions of the reaction can be sufficiently changed to achieve the desired catalytic effect. Ruthenium salts such as those of organic acids can be employed in the practice of this invention to produce the catalyst. In addition to those ruthenium compounds described in the aforementioned Gresham patent, one may employ ruthenium compounds of bidentate ligands, allyl complexes, arene complexes, halides, and alkyl complexes. The choice of ruthenium compounds is varied and not critical to this invention. A number of ruthenium complexes are known to be more stable to the presence of carbon monoxide than other ruthenium compounds and the skilled worker can determine which particular ruthenium compound might take longer to initiate a reaction than other ruthenium compounds. On that basis, one can select for the purposes of convenience the particular ruthenium compound to be utilized in forming the catalyst. However, ruthenium which is associated with an organic molecule or complexed with carbon monoxide or halogen is most readily solubilized so as to provide the ruthenium catalyst of this process.

As characterized above, this process is operated in a homogeneous liquid phase mixture. The process is typically carried out in a solvent for the catalyst and the promoter, when added. Thus, the solvent is a liquid in which the catalyst (presumed to be a ruthenium carbonyl complex) and the added promoter are soluble under the prescribed conditions of the reaction. The solvent may be solid at room temperature but should at least in part be a liquid under the conditions of reaction.

A preferred solvent is a liquid at reaction conditions which is polar or complexes cations. Of the polar solvents, those which have a relatively high dielectric constant are more preferred. As for the solvents which complex cations, the desirable solvents are those which under the reaction conditions have the capacity of complexing cations. As stated previously, the solvent may provide the promoter component. Solvents having a dielectric constant at 25°C. or at its melting temperature, whichever is higher, of greater than 2, are preferred.

*For a discussion of such solvents and their mixtures, see U.S. 4,162,261; 4,188,335; 4,197,253; and German 2,643,971.

Illustrative of suitable polar solvents are, e.g., water, ketones, esters including lactones, amides including lactams, sulfones, sulfoxides, halogenated hydrocarbons, aromatic hydrocarbons, and the like. Illustrative of specific solvents encompassed by the above classes of polar solvents are, for example, aromatic hydrocarbons, e.g., toluene, xylene, naphthalene, alkylnaphthalene, etc.; carboxylic acids such as acetic acid, propionic acid, butyric acid, caproic acid, stearic acid, benzoic acid, cyclohexanecarboxylic acid, etc., see the description of acyl compounds in US patent application Serial No. 971,667; ketones such as acetone, methyl ethyl ketone, cyclohexanone, cyclopentanone, etc.; esters such as methyl acetate, ethyl acetate, propyl acetate, butyl acetate, methyl propionate, ethyl butyrate, methyl laurate, etc.; anhydrides such as phthalic anhydride, acetic anhydride, etc.; lactams such as N-alkyl caprolactam, such as N-methylcaprolactam, N-alkyl pyrrolidinones such as N-methyl pyrrolidinone; cyclic ureas such as N,N'-dimethylimidazolidone; polyols such as ethylene glycol, glycerine, erythritol, polyalkylene glycol containing two to about ten thousand repeating units; lactones such as gamma-butyrolactone; halogenated hydrocarbons such as chlorobenzene, chloroform, methylene chloride, 2,2-dichloropropane; amides such as dimethylformamide, dimethylacetamide, hexamethylphosphoramide; sulfones such as sulfolane, dimethylsulfone, the substituted sulfolanes described in U.S. patent application Serial No. 61,456, filed July 27, 1979; sulfoxides such as dimethylsulfoxide, diphenyl sulfoxide; as well as many others.

Illustrative of suitable complexing solvents are the ethers, cryptands, and the like. Illustrative of specific solvents encompassed by the above classes of complexing solvents are, for example, ethers such as tetrahydrofuran, tetrahydropyran, diethyl ether, 1,2-dimethoxybenzene, 1,2-diethoxybenzene, the mono- and dialkyl ethers of alkylene and polyalkylene glycols, such as ethylene glycol, of 1,2-propylene glycol, of 1,2-butylene glycol, of diethylene glycol, of di-1,2-propylene glycol, of triethylene glycol, of pentaethylene glycol (such as triglyme, tetraglyme and pentaglyme), of di-1,2-butylene glycol, of oxyethylene-oxypropylene glycols, etc., preferably those in which the alkylene group contains 2 and/or 3 carbon atoms in the divalent moiety, such as ethylene and 1,2-propylene; the cryptands such as described in U.S. Patent No. 4,111,975, which description of cryptands, as promoters in that case, is incorporated herein by reference; the crown ethers such as described in U.S. Patent No. 4,162,261, which description of crown ethers, as solvents in that case, is incorporated herein by reference; as well as many others.

An important class of solvents contemplated in the practice of this invention are mixtures of the aforementioned polar solvents and the complexing solvents. Various polar solvents mixed with other polar or complexing solvents are contemplated to provide enhanced results either in terms of rates, selectivity, conversions and/or yields of one or more of the desired products. Which mixtures will achieve what result has not been determined. Combinations of, e.g., sulfolane with crown ethers, lactones, amides or ureas are contemplated as potentially useful. Combinations of, e.g., crown ethers with lactones, amides, and ureas are contemplated as potentially useful.

The promoters suitable in the practice of this process are not a narrowly defined class of materials. They encompass a broad range of inorganic and organic materials. Their effectiveness in some instances can be noted when used in as little an amount which is the least amount for which a measurable promotional effect is seen to an amount wherein the promoter is also a solvent for the reaction. The promoter can serve a dual function by playing the role as the solvent for the reaction. There is no simple way of determining what promoter will function effectively under a given set of reaction conditions. In the typical case, when a promoter exhibits promotional effects on the rate of the reaction, it is present and dissolved in the liquid phase in a range of from about .01 mole to about $10^6$ moles for each atom (gram atomic weight) of ruthenium present in the reaction. More preferred, the promoter is present (even when the solvent used is a promoter) in the liquid phase in a range from about 1 mole to about $10^4$

moles for each atom of ruthenium present in the reaction; most preferably, greater than one mole up to about 1000 moles of the promoter for each atom of ruthenium present and dissolved in the liquid phase.

The promoters include inorganic as well as organic compounds. Illustrative of suitable organic compounds are those containing at least one nitrogen atom or at least one oxygen atom or a combination of such nitrogen and oxygen atoms. The carbon atoms can be part of an acyclic and/or cyclic radical such as aliphatic, cycloaliphatic and aromatic carbon radicals. Usually, the organic promoters contain at least 2 carbon atoms and no more than 40 carbon atoms. The nitrogen atoms are usually in the form of imino (-N=), amino (-N-) and nitrilo (N≡), etc. The oxygen atoms can be in the form of groups such as hydroxyl (aliphatic or phenolic), carboxyl ($-\overset{O}{\overset{\|}{C}}-OH$), carbonyloxy ($-\overset{O}{\overset{\|}{C}}-O$), oxy (-O-), carbonyl ($-\overset{O}{\overset{\|}{C}}-$), etc. The organic promoters may also contain other atoms and/or groups as substituents of the aforementioned radicals such as alkyl, aryl and chloro substituents. The promoter also includes a variety of inorganic compounds such as, for example, inorganic amines and a variety of inorganic metal compounds.

Illustrative of suitable classes of promoters are, for example, any of the following: monoamines and polyamines, including those compounds in which nitrogen forms part of a ring structure;

alkanolamines; acyl compounds including aliphatic, cycloaliphatic and aromatic carboxylic acids, ester derivatives and anhydrides of such acids, usually having no more than 20 carbon atoms; bis(triorgano phosphine)iminium compounds; ketones; ethers; amides; crown ethers; cryptands; hydroxides and salts of various metals including, for example, carboxylates, halides, carbonates, bicarbonates, sulfates and bisulfates of any of the alkali metals, alkaline earth metals as well as of other metals such as iron; as well as many other compounds which can function as promoters or serve as a source for the promoter under reaction conditions.

Illustrative of specific promoters are the following:

Methyl-, ethyl-, isopropyl- and octylamines

Dimethyl-, diisoamyl- and diisobutylamines

Methylethylamine

Trimethyl- and triethylamines

Methyldiethylamine

Triisobutyl- and tridecylamines

1,2-Ethanediamine

1,3-Propanediamine

Diethylenetriamine

Triethylenetetraamine

Tetraethylenepentaamine,

$NH_2CH_2CH_2NHCH_2CH_2NHCH_2CH_2NHCH_2CH_2NH_2$

N,N,N',N'-Tetramethylethylenediamine,

$(CH_3)_2NCH_2CH_2N(CH_3)_2$

p-Phenylenediamine

o-Tolidene

Aniline

1-Naphthyl- and 2-naphthylamines

p-Toluidine

Benzylamine

Diphenylamine

Dimethylaniline

Bis-(1,8)-dimethylaminonaphthalene

Cyclohexylamine

Dicyclohexylamine

Piperidine and N-methylpiperidine

3-Phenylpiperidine

Pyridine and 2-methylpyridine

2,4,6-Trimethylpyridine

2-Dodecylpyridine

2-Aminopyridine

2-(Dimethylamino)pyridine

Quinoline

2-(Dimethylamino)-6-methoxyquinoline

Pyrimidine

1,8-Phenanthroline

Piperazine

N-methyl- and N-ethylpiperazines

2,2'-Bipyridyl and alkyl-substituted 2,2'-bipyridyls

1,4-Diazabicyclo[2.2.2]octane ("triethylenediamine")

Hexamethylenetetraamine

Purine

Isopropanolamine

Diethanolamine

Di-n-propanolamine
Triethanolamine
Triisopropanolamine
Bis(dimethylaminoethyl)ether
N,N-dimethylglycine
N-methyliminodiacetic acid
2-Hydroxypyridine
2-Methoxypyridine
2,6-Dimethoxypyridine
4-Methyl-2-hydroxypyridine
4-Methyl-2,6-dihydroxypyridine
Morpholine
N-methyl- and N-ethylmorpholines
Hexadecylmorpholine
Ethylenedimorpholine
Tetraethylenedimorpholine
Picolinic acid
Nitrilotriacetic acid
2,5-Dicarboxypiperazine
N-(2-hydroxyethyl)-iminodiacetic acid
2,6-Dicarboxypyridine
Ammonia
Hydroxylamine
Hydrazine
Hexamethylphosphoramide
Dimethylformamide
N-Methylpyrrolidinone
Acetic acid
Propionic acid
Butyric acid

2,2,6,6-Tetramethylheptane-3,5-dione, $(CH_3)_3CC(O)CH_2C(O)C(CH_3)_3$

Sulfolane

18-Crown-6

15-Crown-5

Tetrahydrofuran

Diphenylether

Bis(triphenylphosphine)iminium chloride, $[(C_6H_5)_3P]_2N^+Cl^-$

Bis(triphenylphosphine)iminium iodide, $[C_6H_5)_3P]_2N^+I^-$

Cesium formate

Sodium acetate

Sodium sulfate

Potassium carbonate

Potassium bicarbonate

Cesium oxide

Cesium hydroxide

Potassium hydroxide

Magnesium bromide

Calcium iodide

Cesium bromide

Sodium fluoride

Potassium fluoride

Rubidium bromide

Cesium iodide

Rubidium iodide

Potassium iodide

Sodium iodide

Lithium iodide

Lithium bromide

Lithium chloride

Potassium chloride

Lithium diethylamide

Sodium phenyl

Butyllithium

Cobalt diiodide, e.g. $CoI_2.2H_2O$

Tetracarbonyl cobaltate anion, $[Co(CO)_4]^{-1}$

Ferrous iodide, e.g. $FeI_2.4H_2O$

Not all of the above promoters, or for that matter all promoters, will necessarily function effectively in all of the embodiments of the process of this invention. In most cases a degree of selection between the choice of promoter, the amount of ruthenium, the choice of solvent and the reaction parameters will be required to obtain the level of productivity sought.

Because $H_2$ is supplied to the reaction, a hydride of ruthenium can exist in the reaction system. There is no appreciation of the particular role that hydride is playing in the reaction. It is believed that either too much or too little hydrogen present in the reaction will not favor the production of ethylene glycol. In such a case, one can contemplate a role for hydride in the reaction mechanisms occurring.

The relative amounts of carbon monoxide and hydrogen which are initially present in the reaction mixture can be varied over a wide range. In general, the molar ratio of $CO:H_2$ is in the range of from about 40:1 to

about 1:40, suitably from about 20:1 to about 1:20, and preferably from about 10:1 to about 1:10. It is to be understood, however, that molar ratios outside the broadest of these ranges may be employed. Substances or reaction mixtures which give rise to the formation of carbon monoxide and hydrogen under the reaction conditions may be employed instead of mixtures comprising carbon monoxide and hydrogen which are used in preferred embodiments in the practice of the invention. For instance, the product alcohols are contemplated as obtainable by using mixtures containing carbon dioxide and hydrogen. Mixtures of carbon dioxide, carbon monoxide and hydrogen can also be employed. If desired, the reaction mixture can comprise steam and carbon monoxide.

The quantity of catalyst employed is not narrowly critical and can vary over a wide range. In general, the process is desirably conducted in the presence of a catalytically effective quantity of the active ruthenium species which gives a suitable and reasonable reaction rate. Reaction can proceed when employing as little as about 1 x $10^{-6}$ weight percent, and even lesser amounts, of ruthenium based on the total weight of reaction mixture (i.e., the liquid phase mixture). The upper concentration limit can be quite high, e.g., about 30 weight percent ruthenium, and higher, and the realistic upper limit in practicing the invention appears to be dictated and controlled more by economics in view of the cost of ruthenium. Since the rate of conversion of synthesis gas may be dependent upon the concentration of ruthenium employed, higher concentrations achieving higher

rates, then large concentrations may prove to be a most desirable embodiment of this invention. Depending on various factors, such as the promoter (if employed), the partial pressures of carbon monoxide and hydrogen, the total operative pressure of the system, the operative temperature, the choice of solvent, and other considerations, a catalyst concentration of from about $1 \times 10^{-3}$ to about 20 weight percent ruthenium (contained in the complex catalyst) based on the total weight of reaction mixture, is generally desirable in the practice of the invention.

The temperature which may be employed in practicing the process may vary over a wide range of elevated temperatures. In general, the process can be conducted at a temperature between about 50°C. and about 400°C. and higher. Temperatures outside this stated range, though not excluded from the scope of the invention, do not fall within certain desirable embodiments of the invention. At the lower end of the temperature range, and lower, the rate of reaction to desired products becomes markedly slow. At the upper temperature range, and beyond, catalyst, solvent, or promoter instability may occur. Notwithstanding these factors, reaction will continue and the alcohols and/or their derivatives will be produced. Preferred temperatures are between about 100°C. and about 350°C., and most desirably between about 150°C and about 300°C.

The process is suitably effected over a wide superatmospheric pressure range. At pressures in the direction of and below about $35.15 \times 10^5$ Pa (500 psia or 35.15 $kg/cm^2$) the rate of desired product formation is quite slow, and consequently, relatively faster reaction rates and/or higher conversions to the desired products can be obtained by employing higher pressures, e.g., pressures of at least about $70.31 \times 10^5$ Pa (1,000 psia or 70.31 $kg/cm^2$). Pressures as high as $1,406 \times 10^5$ to $3,515 \times 10^5$ Pa (20,000 to 50,000 psia or 1,406 to 3,515 $kg/cm^2$) can be employed but there is no apparent advantage in using such pressures, and any advantage that could be reasonably contemplated would be easily offset by the very unattractive plant investment outlay required for such high pressure equipment and the costs associated with such high pressure operations. Therefore, the upper pressure limitation is approximately $1,054.6 \times 10^5$ Pa (15,000 psia or 1,054.6 $kg/cm^2$); pressures below about $703.1 \times 10^5$ Pa (10,000 psia or 703.1 $kg/cm^2$) result in significant cost advantages which are associated with lower pressure equipment requirements and operating costs. A suitable pressure range is from about $35.15 \times 10^5$ Pa (500 psia or 35.15 $kg/cm^2$) to about $878.84 \times 10^5$ Pa (12,500 psia or 878.84 $kg/cm^2$). The pressures referred to above represent the total pressure of hydrogen and carbon monoxide.

The process is effected for a period of time sufficient to produce the desired alcohol products and/or derivatives thereof. In general, the residence time to produce the desired products can vary from minutes to a number of hours, e.g., from a few minutes to 24 hours, and longer. It is readily appreciated that the residence period (time) will be influenced to a significant extent by the reaction temperature, the concentration and choice of promoter and ruthenium source, the total gas pressure and the partial pressure exerted by its components, the concentration and choice of solvent, and other factors. The synthesis of the desired product(s) by the reaction of hydrogen with carbon monoxide is suitably conducted under operative conditions which give reasonable reaction rates and/or conversions.

The process can be executed in a batch, semi-continuous, or continuous fashion. The reaction can be conducted in a single reaction zone or a plurality of reaction zones, in series or in parallel, or it may be conducted intermittently or continuously in an elongated tubular zone or series of such zones. The material of construction should be such that it is inert during the reaction and the fabrication of the equipment should be able to withstand the reaction temperature and pressure. The reaction zone can be fitted with internal and/or external heat exchanger(s) to thus control undue temperature fluctuations, or to prevent any possible "runaway" reaction temperatures due to the exothermic nature of the reaction. In preferred embodiments of the invention, agitation means to vary the degree of mixing of the reaction mixture can be suitably employed. Mixing induced

by vibration, shaker, stirrer, rotatory, oscillation, ultrasonic, etc., are all illustrative of the types of agitation means which are contemplated. Such means are available and well-known to the art. The catalyst precursor may be initially introduced into the reaction zone batchwise, or it may be continuously or intermittently introduced into such zone during the course of the synthesis reaction. Means to introduce and/or adjust the reactants, either intermittently or continuously, into the reaction zone during the course of the reaction can be conveniently utilized in the process especially to maintain the desired molar ratios of and the partial pressures exerted by the reactants.

In order to describe this invention with a greater degree of particularity, reference is made, for the purposes of illustration only, to the accompanying drawing which depicts a schematic flowsheet of a continouous operating unit for the practice of the process of this invention. Referring to the drawing, reactor 1, which is a back-mixed stirred reactor, has thereabout a jacket 3 through which is flowed a heat transfer fluid for the purposes of maintaining temperature control. Reactor 1 contains stirrer 5 for the purposes of maintaining uniform distribution of product and solution in the reactor during the course of the reaction. The reactor 1 is fabricated from 316 stainless steel of sufficient strength to withstand up to $2{,}108 \times 10^5$ Pa (30,000 psi). To the reactor 1 is supplied, through line 19, recycle and synthesis gas. A carbon monoxide feed stream 6 and hydrogen feed stream are mixed in the desired ratio using a metering system

(not shown) which allows the composition of gas to be varied from pure $H_2$ to pure CO. The synthesis gas feed is passed through compressor 9 to produce in line 11 a synthesis gas stream at the desired pressure. This gas stream in line 11 is combined with a liquid recycle stream of solvent from line 13 and introduced via line 15 into a pre-heater 17 to heat the mixture of solvent recycle and synthesis gas in line 15 to a temperature very close to the reaction temperature utilized within reactor 1. Methanol, or ethanol, is supplied to the heated feed stream from preheater 17 through line 16 in the concentration desired for the purpose of decreasing the net production of the particular alcohol, as described above. The combined feed composition is passed through line 19 into the reactor 1.

The effluent from the reactor, which is a mixture of gas and liquid containing the products of the reaction, unreacted synthesis gas, and solvent, passes through line 21 to cooler 23, where the stream is reduced to a temperature of about 100-150°C., and thereafter through line 25 to pressure reducing valve 27 where the pressure of effluent entering hold tank 28 is reduced to about 10% of the reaction pressure. Hold tank 28, which contains stainless steel packing rings to enhance gas-liquid contact, serves to resolubilize volatilized ruthenium complex compounds into the liquid stream. The effluent from hold tank 28 is fed to high pressure separator 29. Separator 29 serves the function of separating substantial amounts of the liquid products and solvent from the effluent stream. A portion of unreacted synthesis gas dissolved in the liquid product

will also be separated therefrom in separator 29. A stream of essentially gaseous material comprising some methanol and other low boiling components as well as a significant part of the synthesis gas in the effluent stream from line 25 is taken off the top of separator 29 through line 32. The gas stream from line 32 is passed through a throttle valve 31 which controls the pressure in separator 29. Valve 35 in line 33 serves to control the liquid level within the separator 29. The effluent of line 32 is thereafter fed to a low pressure separator 37. High pressure separator 29, typically, is operated at a pressure which is approximately 10% that contained within reactor 1, whereas low pressure separator 37 is operated at about atmospheric pressure to somewhat above atmospheric pressure. Typically, low pressure separator 37 is operated at as low a pressure as possible, taking into consideration the desire to transport the liquid streams fed therein to stripper 53. The liquid stream which exits from the bottom of high pressure separator 29 is carried via line 33 through throttle valve 35 to low pressure separator 37 and the liquid is collected at the bottom of separator 37.

The gases vented from low pressure separator 37 are taken by way of line 39 through heat exchanger 45 to reduce the temperature of the stream and enhance further removal of liquid products and then to receiver 47. Synthesis gas and uncondensed products are removed from receiver 47 through line 49 and pressure control valve 52, and pass through a chilled methanol scrubber 48 prior to being vented to the atmosphere. Scrubber 48 serves to recover volatilized ruthenium compounds contained

in the gas stream from receiver 47. Typically, such vented gases are predominantly the non-condensable gases as well as very small amounts of methanol, ethanol and methyl formate. The liquid product recovered in receiver 47 is primarily methanol which is drawn off line 51 and can be recycled back to reactor 1 by connecting line 51 with line 16.

The liquid collected in separator 37 is withdrawn therefrom through line 41 and throttle valve 43 and passed to the upper portion of gas stripper 53. Stripper 53, surrounded by steam jacketing 55, contains therein a stainless steel wire mesh packing of the type which creates a very low pressure drop within the column. The liquid product from the low pressure separator 37 is stripped in stripper 53 with synthesis gas which is circulated through stripper 53 in a continuous gas recycle loop with makeup quantities of gas being provided through line 70. Synthesis gas is fed into the lower end of the stripper 53 through line 59 after having been heated in heat exchanger 58 and countercurrently strips the more volatile products contained in the liquid stream introduced through line 41. Stripping gas and vapor products are removed from the overhead of the stripper 53 through line 57, are cooled in condenser 61, and are then collected in product receiver 63. The liquid products collected in receiver 63 are predominantly methanol, ethanol and ethylene glycol, which are separated from one another by simple distillation, after withdrawal from receiver 63 through line 65. The gases and small amount of vapor products in receiver 63 are withdrawn through line 69 to

recycle compressor 71 and are then passed to stripper 53 to complete the continuous gas loop.

The stripped liquid recovered from the bottom of stripper 53 via line 67 is carried to a collection tank 73 from which it is fed via line 75 into solvent pump 77 for recycling to reactor 1 through line 13 after being admixed with the synthesis gas in line 11 as previously described.

In a typical operation of the aforementioned process, when the solvent employed is extremely volatile, the solvent will oftentimes be carried over with some of the most volatile products and be collected with them in, for example, receiver 47, or product receiver 63. Obviously, in the optimum operation of the process it is desirable that all of the solvent be continuously recycled without the need to be separated in an isolated step independent of the cyclic mode of the process.

In the most favorable utilization of the process of this invention, the decrease in net production of alcohol is effected when the process is operated continuously, and most desirably, in a cyclic mode. In a cyclic mode, the effluent from the reactor will contain the solvent as well as product and go through a multiplicity of separation steps, as herein described, so as to allow the recovery of products, preferably, independent of the solvent. Also contained within the solvent is the dissolved ruthenium values which are utilized in effecting the catalytic reaction. Because of the unique stability of ruthenium in such a system, very little, if any of it, is lost over an extended operation of a cyclic process. Therefore, one

may repeatedly recycle the ruthenium with very little diminution in catalyst concentration and rate of product production.

The alcohol which is provided to the reactor for the purpose of decreasing the net production of such alcohol in accordance with the practice of the invention can be introduced in many ways. For example, the alcohol can be formed in situ or can be introduced from an independently derived alcohol source. It may also be independently introduced by recycling recovered alcohol when the process is on stream, or it may be introduced to the reaction with the solvent that is recycled by simply not separating the alcohol from the solvent during the separation phase of the process. How one recycles the alcohol to the reaction is quite unimportant in the practice of this invention.

The amount of alcohol which one provides in the reaction in which ethylene glycol is being formed is, from a minimum standpoint, that effective amount which achieves a detectable amount of decrease in the net production of the particular alcohol. It is anticipated that the maximum amount of alcohol employed in the process is that amount which will achieve a virtual elimination of the net production of the particular added alcohol although amounts greater than this amount may be added for other beneficial effects. The amount of alcohol which is added is not a narrowly defined amount because there are a number of factors which will contribute to the degree of decrease in net production. For example, such factors as temperature, pressure,

solvent utilized, amount of active ruthenium catalyst present, residence time, and like considerations, will aid in defining the parameters under which one would operate for decreasing the net production of alcohol. In a typical case, the amount of alcohol one might add for the purpose of decreasing the net production of alcohol can be as little as .2 wt. % of the liquid phase which comprises the reaction medium and as much as about 50 wt. %. In most cases the amount of alcohol added for the purpose of decreasing the net production of alcohol will be from about 1 wt. % to about 30 wt. % and most preferably from about 2 wt. % to about 20 wt. %.

Though this invention has been described in considerable detail, the following examples are provided for the purpose of giving more specific illustration of the invention contemplated herein. It is not intended that these examples should in any way act to limit the scope of this invention.

EXPERIMENTAL PROCEDURE

The following procedure was employed in the examples hereinafter discussed:

A 150 milliliter capacity stainless steel reactor capable of withstanding pressures of up to $3000 \times 10^5$ Pa (3,000 atmospheres) was charged with a mixture of solvent, methanol (or ethanol), ruthenium as triruthenium dodecacarbonyl and Lewis base promoter, as indicated in the examples below. The reactor was sealed and charged with carbon

BAD ORIGINAL

monoxide to a pressure of 36.19 x $10^5$Pa (500 psig or 36.19 kg/$cm^2$). In some cases the gaseous contents of the reactor are vented to remove oxygen. In these cases the reactor is then repressurized to about 36.19 x $10^5$Pa (500 psig). (This venting procedure may be repeated if desired.)

Heat was then applied to the reactor and its contents, initially at about 55°C or as otherwise indicated; when the temperature of the mixture inside the reactor reached the designated reaction temperature, as measured by a suitably placed thermocouple, addition of carbon monoxide and hydrogen ($H_2$:CO equals the designated mole ratio) was made to bring the pressure to the specified reaction pressure. The temperature was maintained at the desired value for the reported time period. During this period of time, additional carbon monoxide and hydrogen were added whenever the pressure inside the reactor dropped by more than about 36.19 x $10^5$Pa (500 psig). With these added repressurizations the pressure inside the reactor was maintained at the reaction pressure plus or minus about 36.19 x $10^5$Pa (500 psig) over the entire reaction period.

After the reaction period, the reaction vessel was cooled to room temperature, the reaction vessel vented and the reaction products removed. Analysis of the reaction mixture was made by gas

BAD ORIGINAL

chromatographic methods.

The various rates set forth in the following examples are average rates for the particular product and are determined by measuring the net production of product for the reaction period and assuming a nominal reaction volume of 75 milliliters and are expressed as moles per liter of solution per hour.

EXAMPLES 1-10

According to this invention the following examples were carried out according to the Experimental Procedure to demonstrate the decrease in net production of added alcohol. Table I sets forth comparative examples 1-5 and examples 5-10 which are examples of the invention wherein the net production of methanol is decreased by providing methanol to the reaction system.

EXAMPLES 11-16

Comparative example 11 and examples 12-16 were carried out according to the above-described experimental procedure to demonstrate the decrease in net production of alcohol (methanol (Examples 12-14) or ethanol (Examples 15 and 16)) when alcohol is provided to the reaction system. These results are set forth in Table II.

BAD ORIGINAL

TABLE I (1)

| Examples | Added Methanol (g) | t, hr | Ethylene Glycol Grams | Ethylene Glycol Rate | Methanol Grams | Methanol Rate | Ethanol Grams | Ethanol Rate | Notes |
|---|---|---|---|---|---|---|---|---|---|
| 1 | 10 | 0.08 | - | - | 9.41 | - | - | - | 2,3 |
| 2 | 10 | 0.08 | - | - | 10.00 | - | - | - | 3,4 |
| 3 | -0- | 1.0 | 3.69 | .81 | 5.93 | 2.47 | .56 | .12 | 2 |
| 4 | -0- | 1.0 | 5.19 | 1.13 | 4.91 | 2.06 | .67 | .20 | 2 |
| 5 | -0- | 1.0 | 5.81 | 1.25 | 6.09 | 2.54 | .79 | .24 | 4 |
| 6 | 10 | 1.0 | 3.85 | .82 | 9.72 | 0.06 | 1.52 | .45 | 2,5 |
| 7 | 10 | 1.0 | 5.24 | 1.13 | 10.13 | .05 | 1.02 | .30 | 2 |
| 8 | 10 | 1.0 | 5.85 | 1.26 | 10.85 | .35 | 1.20 | .35 | 2 |
| 9 | 10 | 1.0 | 5.17 | 1.11 | 10.63 | .26 | 1.64 | .47 | 4 |
| 10 | 10 | 1.0 | 5.01 | 1.08 | 11.51 | .63 | 1.67 | .48 | 4 |

1. Conditions: 75 ml sulfolane solvent, 30 mmoles Ru, 120 mmoles KI, $878.84 \times 10^5$ Pa (12,500 psi) ($H_2$:CO of 1:1) 200°C.
2. Two pre-run gas purges with $36.19 \times 10^5$ Pa (500 psi) CO at 55°C.
3. No Ru or KI added.
4. No pre-run purges under pressure; reaction system was flushed with $N_2$ under $10^5$ Pa (1 atm.) pressure at 25°C.
5. It is believed that a portion of added methanol was converted to other compounds.

TABLE II[1]

| Examples | Added Alcohol, | Grams | t, hr | Ethylene Glycol Grams | Ethylene Glycol Rate | Methanol Grams | Methanol Rate | Ethanol Grams | Ethanol Rate |
|---|---|---|---|---|---|---|---|---|---|
| 11 | - | -0- | .58 | 1.32 | .57 | 7.32 | 5.96 | .76 | .44 |
| 12 | MeOH | .50 | .48 | 1.39 | .66 | 6.44 | 5.13 | .61 | .40 |
| 13 | MeOH | 1.0 | .48 | 1.35 | .67 | 7.06 | 5.23 | .77 | .50 |
| 14 | MeOH | 2.0 | .52 | 1.39 | .65 | 7.75 | 4.64 | .81 | .50 |
| 15 | EtOH | .50 | .60 | 1.69 | .70 | 7.44 | 5.92 | 1.27 | .37 |
| 16 | EtOH | 1.0 | .48 | 1.32 | .65 | 5.59 | 5.21 | 1.22 | .13 |

(1) Conditions: 75 ml 18-crown-6 solvent, 6 mmoles Ru, 18 mmoles KI, $878.84 \times 10^5$ Pa (12,500 psi) ($H_2$:CO of 1:1) 230°C.

EXAMPLES 17-26

Comparative examples 21, 23 and 25 and examples 17-20, 22, 24 and 26 were carried out according to the above-described Experimental Procedure and the reaction conditions set forth in Table III. Examples 17-26 show a decrease in net production of added alcohol.

TABLE III

| Example | Added Methanol (g) | time (hr.) | Ethylene Glycol Grams | Ethylene Glycol Rate | Methanol Grams | Methanol Rate | Ethanol Grams | Ethanol Rate |
|---|---|---|---|---|---|---|---|---|
| 17 (1) | 1 | 0.23 | 1.93 | 1.78 | 7.45 | 11.50 | 0.48 | 0.60 |
| 18 (1) | 10 | 0.23 | 1.79 | 1.65 | 13.50 | 6.43 | 1.12 | 1.39 |
| 19 (1) | .85 | 0.25 | 1.96 | 1.68 | 7.38 | 10.89 | 0.48 | 0.57 |
| 20 (1) | 9.5 | 0.23 | 1.69 | 1.48 | 12.66 | 5.69 | 1.03 | 1.28 |
| 21 (2) | - | 0.32 | 0.75 | 0.51 | 4.72 | 6.23 | 0.53 | 0.49 |
| 22 (3) | 20 | 0.53 | 0.53 | 0.21 | 17.47 | -1.98 (7) | 1.64 | 0.89 |
| 23 (4) | - | 0.37 | 1.31 | 0.77 | 3.62 | 4.12 | 0.16 | 0.14 |
| 24 (4) | 10 | 0.42 | 0.34 | 0.18 | 11.52 | 1.52 | 0.79 | 0.49 |
| 25 (5) | - | 0.70 | 1.40 | 0.43 | 3.00 | 1.81 | 0.53 | 0.22 |
| 26 (6) | 15 | 1.25 | 0.98 | 0.17 | 4.45 | -3.18 (7) | 2.57 | 0.60 |

(1) Conditions: 15 mmole ruthenium, 90 mmole NaI, 878.84 x $10^5$ Pa (12,500 psig) ($H_2$:CO of 1:1) in 75 ml. sulfolane, 230°C.

(2) Conditions: 45 mmole ruthenium, 22.5 mmoles NaI, 67.5 ml. N-methyl pyrrolidone, 7.5 ml. of 18-Crown-6, 434 x $10^5$ Pa (6000 psig) ($H_2$:CO of 1:1), 230°C.

(3) Conditions: 45 mmole ruthenium, 22.5 mmoles NaI, 37.5 ml. of N-methyl pyrrolidone, 37.5 ml. of 18-Crown-6, 434 x $10^5$ Pa (6000 psig). ($H_2$:CO of 1:1), 230°C.

TABLE III (Continued)

(4) Conditions: 45 mmoles ruthenium, 202.5 mmoles NaI, 37.5 ml. N-methyl pyrrolidone, 37.5 ml. of 18-Crown-6, 434 x $10^5$Pa (6000 psig). ($H_2$:CO of 1:1) 200°C.

(5) Conditions: 45 mmoles Ru, 112.5 mmoles NaI, 52.5 ml. N-methyl pyrrolidone, 22.5 ml. of 18-Crown-6, 434 x $10^5$Pa (6000 psig). ($H_2$:CO of 1:1).

(6) Conditions: Same as set forth in (6) except that 37.5 ml. of N-methyl pyrrolidone and 37.5 ml. of 18-Crown-6 were employed.

(7) A negative (-) rate to methanol indicates that some portion of the added methanol was converted to other compounds.

CLAIMS

1. A process for selectively making the alcohol products methanol, ethanol, ethylene glycol and derivatives thereof by reacting carbon monoxide and hydrogen in the presence of a ruthenium carbonyl complex at a temperature of between about 50°C and 400°C and a pressure of between about 35.15 x $10^5$Pa (500 psia or 35.15 kg/$cm^2$) and about 1,054.6 x $10^5$Pa (15,000 psia or 1,054.6 kg/$cm^2$) for a sufficient period of time to produce said products characterized in that an effective amount of one or more of said alcohol products are provided to the process such that the net production of the alcohol product corresponding to said provided alcohol product is decreased.

2. The process of claim 1 for selectively making the alcohol products methanol, ethanol, ethylene glycol, and derivatives thereof by providing an effective amount of one or more of said alcohol products to the process, wherein said process comprises:

(a) establishing and maintaining a solvent-containing liquid phase comprising solubilized ruthenium carbonyl complex in which the solvent has a dielectric constant of at least 2, determined at 25°C. or at its melting point, whichever is higher;

(b) supplying hydrogen and carbon monoxide in said liquid phase;

(c) providing an effective amount of one or more of said alcohol products to said liquid phase; and

(d) maintaining said liquid phase for a sufficient period of time at a temperature and pressure which causes said hydrogen and carbon monoxide to react to produce such products, said temperature is between about 50°C. and 400°C. and said pressure is between about 35.5 x $10^5$Pa (500 psia or 35.5 kg/$cm^2$) and 1,054.6 x $10^5$Pa (15,000 psia or 1,054.6 kg/$cm^2$), such that the net production of the alcohol product corresponding to the alcohol product provided in step (c) is decreased.

3. The process of claim 1 or 2 wherein the alcohol of step (c) is methanol, ethanol or a mixture of methanol and ethanol.

4. The process of claim 1 or 2 wherein said alcohol of step (c) is provided in an amount from about .2 percent by weight to about 50 percent by weight, preferably in an amount from about 1 percent by weight to about 30 percent by weight and more preferably in an amount from about 2 percent by weight to about 20 percent by weight.

5. The process of claim 1 wherein a process is carried out in the liquid phase.

6. The process of claim 2 wherein a promoter of the reaction is provided to the liquid phase, preferably the said promoter being a Lewis base containing compound.

7. The process of claim 5 wherein the solvent acts as a promoter, preferably the said solvent being a Lewis base containing compound.

8. The process of claim 5 wherein the solvent is polar and is preferably water, N-methyl pyrrolidone or a mixture of a crown ether and N-methyl pyrrolidone.

9. The process of claim 5 wherein the solvent complexes ions and is preferably sulfolane or a crown ether.

10. The process of claim 1 or 2 wherein the temperature is between about 100°C. and about 350°C.

11. The process of claim 1 or 2 wherein the pressure is the total pressure of hydrogen and carbon monoxide supplied to said process.

12. The process of claim 6 wherein the promoter is an alkali metal halide, which is preferably an alkali metal iodide and which is more preferably selected among sodium iodide, lithium iodide, potassium iodide or cesium iodide.

13. The process of claim 2 wherein the carbon monoxide and hydrogen are continuously supplied to the liquid phase and product is removed continuously from said liquid phase in combination with unreacted carbon monoxide and hydrogen, preferably wherein unreacted carbon monoxide and hydrogen are recycled to the liquid phase and more preferably wherein a promoter of the reaction is provided to the liquid phase.

14. The process of claim 6 wherein the amount of promoter provided to the reaction is that amount which achieves a measurable promotional effect and this amount preferably ranges from about 0.1 mole to about $10^6$ moles for each gram atom of ruthenium present.

15. The process of claim 2 wherein an alcohol product of step (c) is provided directly to the liquid phase and preferably wherein the amount of alcohol provided in step (c) achieves a measurable decrease in the net production of said alcohol.

16. In a process for selectively making the alcohol products methanol, ethanol, ethylene glycol and derivatives thereof, directly from the reaction of hydrogen and carbon monoxide, wherein said process comprises;

(a) establishing and maintaining a solvent-containing liquid phase comprising solubilized ruthenium carbonyl complex in which the solvent has a dielectric constant of at least 2, determined at 25°C or at its melting point, whichever is higher;

(b) supplying hydrogen and carbon monoxide in said liquid phase; and

(c) maintaining said liquid phase for a sufficient period of time at a temperature and pressure which causes said hydrogen and carbon monoxide to react to produce such products, said temperature is between about 50°C. and 400°C. and said pressure is between about $35.15 \times 10^5$Pa (500 psia or 35.15 $kg/cm^2$) and $1,054 \times 10^5$Pa (15,000 psia or 1,054.6 $kg/cm^2$);

the improvement characterized in that it comprises providing to said process an effective amount of one or more of said alcohol products methanol, ethanol and ethylene glycol such that the net production of alcohol product corresponding to the alcohol provided to the process is decreased, preferably one or more of said alcohol products being provided directly to the liquid phase and more preferably in an amount from about 0.2 percent by weight to about 50 percent by weight.

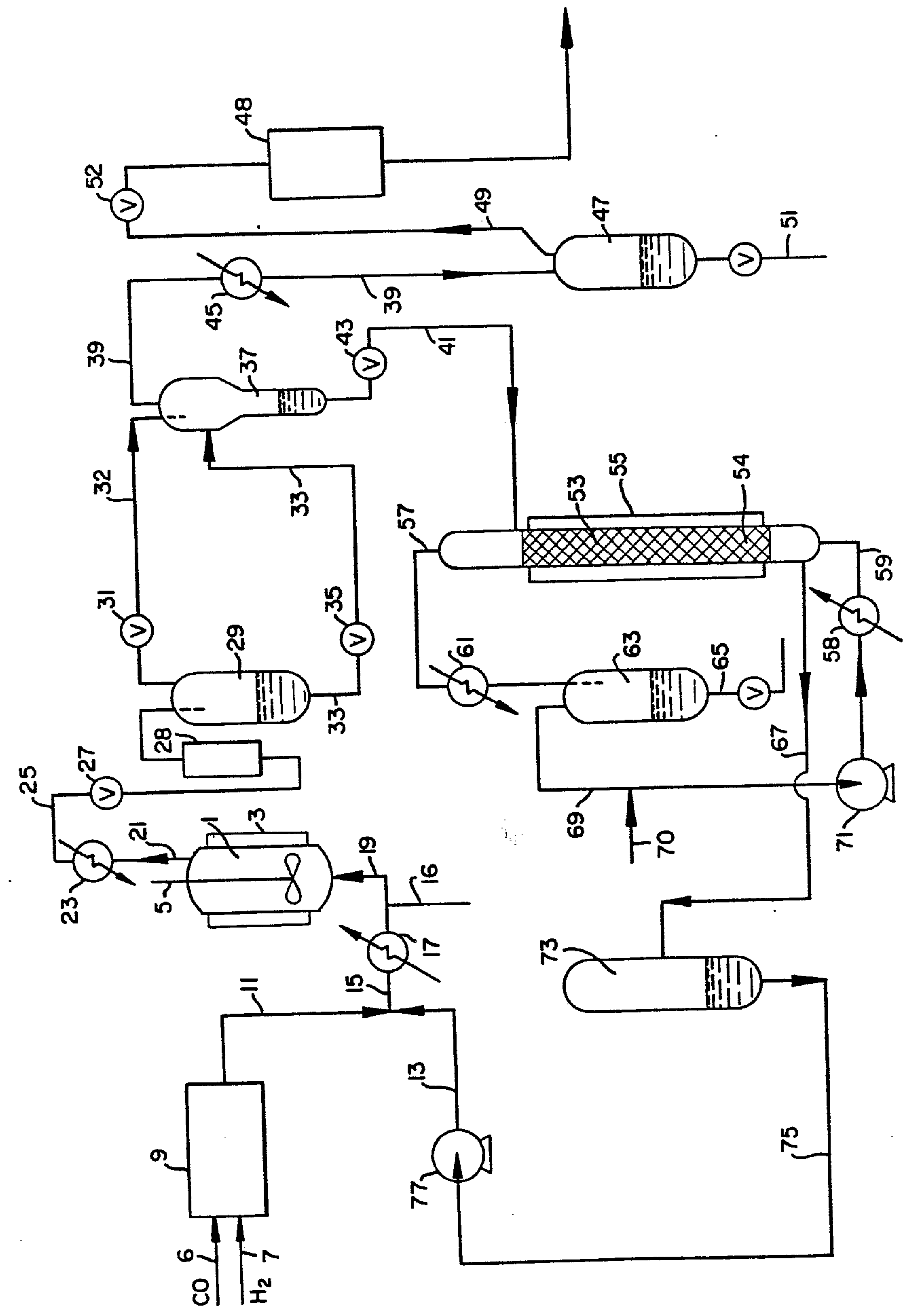
CO
H2

0055668

European Patent Office

# EUROPEAN SEARCH REPORT

Application number
EP 81 40 2065

## DOCUMENTS CONSIDERED TO BE RELEVANT

| Category | Citation of document with indication, where appropriate, of relevant passages | Relevant to claim |
|---|---|---|
| Y | EP - A - 0 006 634 (UNION CARBIDE)<br>* Claim 1; pages 33,34, table I, examples 1,2,5,6,11-13 *<br>-- | 1,2,4-16 |
| Y | EP - A - 0 013 008 (UNION CARBIDE)<br>* Claims *<br>-- | 1,2,4-16 |
| AD | GB - A - 2 024 811 (TEXACO DEVELOPMENT)<br>* Claim 1; page 3, column 1, lines 11-29 *<br>-- | 1-3,5,10 |
| AD | US - A - 4 170 605 (R.C. WILLIAMSON)<br>* Claim 1, column 3, lines 40-45 *<br>-- | 1-3,5,6,10 |
| A | FR - A - 2 234 257 (UNION CARBIDE)<br>* Claims 1,2; page 11, line 23-page 12, line 5; page 16, lines 23-27; page 3, lines 1-5 * | 1-3,5 |
| AD | & US - A - 3 940 432<br>---- | |

CLASSIFICATION OF THE APPLICATION (Int. Cl. 3)

C 07 C 31/04
31/08
31/20
29/15

TECHNICAL FIELDS SEARCHED (Int.Cl. 3)

C 07 C 29/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 10-03-1982 | WRIGHT |

EPO Form 1503.1 06.78